(19) Europäisches Patentamt
European Patent Office
Office européen des brevets

(11) Veröffentlichungsnummer: **0 411 456 A1**

(12) **EUROPÄISCHE PATENTANMELDUNG**

(21) Anmeldenummer: **90114237.2**

(51) Int. Cl.5: **C07D 233/06**

(22) Anmeldetag: **25.07.90**

(30) Priorität: **01.08.89 DE 3925423**

(43) Veröffentlichungstag der Anmeldung: **06.02.91 Patentblatt 91/06**

(84) Benannte Vertragsstaaten: **BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Ebel, Klaus, Dr.**
**Asternstrasse 14**
**D-6704 Mutterstadt(DE)**
Erfinder: **Schroeder, Juergen, Dr.**
**Eichendorffstrasse 7**
**D-6806 Viernheim(DE)**
Erfinder: **Dockner, Toni, Dr.**
**Grossgasse 6**
**D-6701 Meckenheim(DE)**
Erfinder: **Krug, Herbert**
**Mussbacher Strasse 49**
**D-6700 Ludwigshafen(DE)**

# (54) Verfahren zur Herstellung von 2-Imidazolinen.

(57) Verfahren zur Herstellung von 2-Imidazolinen der allgemeinen Formeln Ia und Ib

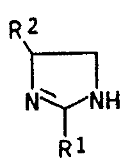

(Ia)

bzw.

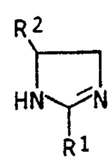

(Ib)

in denen $R^1$ $C_1$- bis $C_8$-Alkyl, $C_7$- bis $C_{12}$-Aralkyl oder Phenyl und $R^2$ Wasserstoff oder $C_1$- bis $C_8$-Alkyl bedeuten, durch Umsetzung von 1,2-Diaminen der allgemeinen Formel II

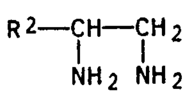

(II),

in der $R^2$ die obengenannte Bedeutung hat, mit Carbonsäuren der allgemeinen Formel III
$R^1$-$CO_2H$ (III), in der $R^1$ die obengenannte Bedeutung besitzt, in einem Verhältnis von 0,8 bis 1,2 mol Ausgangsstoff II je Mol Ausgangsstoff III, bei Temperaturen von 250 bis 400 °C in der Gasphase an Katalysatoren aus γ-Aluminiumoxid und/oder Siliciumoxid, welche gegebenenfalls mit Phophorsäure dotiert sind, umgesetzt und indem man die Reaktion im Festbett durchführt.

EP 0 411 456 A1

## VERFAHREN ZUR HERSTELLUNG VON 2-IMIDAZOLINEN

Die vorliegende Erfindung betrifft ein verbessertes Verfahren zur Herstellung von 2-Imidazolinen durch Umsetzung von 1,2-Diaminen mit Carbonsäuren in der Gasphase bei einer Temperatur von 250 bis 400 °C in Gegenwart eines Festbettkatalysators von $\gamma$-Aluminiumoxid und/oder Siliciumdioxid.

Aus der DE-A-2 512 513 ist ein Verfahren zur Herstellung von 2-Imidazolinen durch Umsetzung von 1,2-Diaminen mit Nitrilen in Gegenwart von Polysulfiden als Katalysatoren bekannt. Nachteile des Verfahrens sind die Bildung von schwefelhaltigen, schwer abtrennbaren Nebenprodukten und die Bildung äquimolarer Mengen an Ammoniak.

Aus der DE-A-2 615 886 ist ein Verfahren zur Herstellung von 2-Imidazolinen durch Umsetzung von Alkancarbonsäuren mit einem Überschuß an 1,2-Diaminen bei Temperaturen unterhalb 160 °C unter Abtrennung von Wasser bekannt. Nachteile des Verfahrens sind die geringen Raum-Zeit-Ausbeuten sowie die benötigte Menge von mindestens 2 mol Diamin je Mol Alkancarbonsäure.

Aus der EP-A-12 371 ist ein Verfahren zur Herstellung von 2-Imidazolinen durch Umsetzung von 1,2-Diaminen mit Nitrilen oder Carbonsäuren bzw. Carbonsäurenderivaten bekannt. Die Umsetzung erfolgt in der Gasphase, in Gegenwart eines unter Reaktionsbedingungen inerten Trägergases, bei einer Temperatur von 300 bis 380 °C in Gegenwart von $\gamma$-Aluminiumoxid und/oder Siliciumoxid, gegebenenfalls in Gegenwart von Phosphorsäure, in der Wirbelschicht. Der gasförmige Reaktoraustrag wird kondensiert und anschließend destilliert. Dieses Verfahren erschien noch verbesserungswürdig.

Der vorliegenden Erfindung lag daher die Aufgabe zugrunde, den zuvor genannten Nachteilen abzuhelfen.

Demgemäß wurde ein verbessertes Verfahren zur Herstellung von 2-Imidazolinen der allgemeinen Formeln Ia und Ib

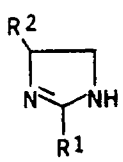

(Ia)

bzw.

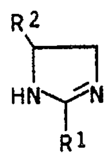

(Ib)

in denen $R^1$ $C_1$- bis $C_8$-Alkyl, $C_7$- bis $C_{12}$-Aralkyl oder Phenyl und $R^2$ Wasserstoff oder $C_1$- bis $C_8$-Alkyl bedeuten, durch Umsetzung von 1,2-Diaminen der allgemeinen Formel II

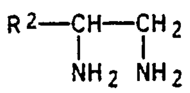

(II),

in der $R^2$ die obengenannte Bedeutung hat, mit Carbonsäuren der allgemeinen Formel III

$R^1$-$CO_2H$ (III), in der $R^1$ die obengenannte Bedeutung besitzt, in einem Verhältnis von 0,8 bis 1,2 mol Ausgangsstoff II je Mol Ausgangsstoff III, bei Temperaturen von 250 bis 400 °C in der Gasphase an Katalysatoren aus $\gamma$-Aluminiumoxid und/oder Siliciumoxid, welche gegebenenfalls mit Phophorsäure dotiert sind, gefunden, welches dadurch gekennzeichnet ist, daß man die Reaktion im Festbett durchführt.

Vorteilhaft wird der Reaktoraustrag gasförmig, bei einer Temperatur oberhalb des Siedepunktes des 2-Imidazolins, in eine kontinuierliche Trennkolonne leitet und das Reaktionswasser sowie nicht umgesetzte Ausgangsstoffe bei einem Druck von 0.01 bis 1 bar, vorteilhaft bei einem Druck von 0.05 bis 0.2 bar, über Kopf abdestilliert.

Die Umsetzung kann durch folgende allgemeine Reaktionsgleichung beschrieben werden.

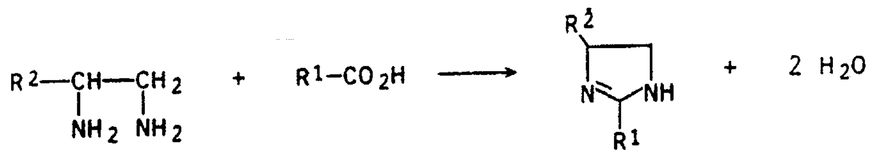

(II) (III) (I)

Die Ausgangsstoffe werden miteinander in einem Verhältnis von 0,8 bis 1,2, insbesondere 0,9 bis 1,1 mol 1,2-Diamin II je Mol Carbonsäure III umgesetzt.

Als 1,2-Diamin II eignen sich Diamine mit 2 bis 20, vorzugsweise 2 bis 10 Kohlenstoffatomen wie 1,2-Diaminoethan, 1,2-Diaminopropan, 1,2-Diamino-n-butan, 1,2-Diamino-n-pentan, 1,2-Diamino-n-hexan, 1,2-Diamino-n-heptan, 1,2-Diamino-n-octan, 1,2-Diamino-n-nonan.

Als Carbonsäuren III eignen sich Carbonsäuren mit 2 bis 20, vorzugsweise 2 bis 10 Kohlenstoffatomen wie Essigsäure, Propionsäure, Buttersäure, 2-Methylpropansäure, n-Hexansäure, 2-Ethylbutansäure, 2-Methylbutansäure, 2,2-Dimethylpropansäure, n-Pentansäure, 2-Methylbutansäure, 2-Methylhexansäure oder entsprechende Gemische wie die bei der Herstellung von natürlichen oder synthetischen Fettsäuren erhaltenen Gemische. Solche Gemische fallen z.B. durch Fettspaltung, durch Paraffinoxidation oder durch die Oxosynthese aus Olefinen, Kohlenoxid und Wasser an.

Die Umsetzung wird bei einer Temperatur von 250 bis 400 °C bei einem Druck von 0,01 bis 1 bar durchgeführt. Temperatur und Druckbedingungen werden so gewählt, daß die Reaktion in der Gasphase stattfindet. Die Umsetzung wird in Gegenwart von Siliciumdioxid und/oder $\gamma$-Aluminiumoxid durchgeführt. Man verwendet in der Regel 10 bis 30 mol 1,2-Diamin II pro Stunde und Liter Katalysator. Als Zusatzkatalysator wird gegebenenfalls Phosphorsäure verwendet, vorteilhaft in einer Menge von 0,01 bis 1, insbesondere 0,01 bis 0,1 Gew.-%, bezogen auf das 1,2-Diamin II. Zweckmäßig sind eine Menge von 1 bis 30, insbesondere 5 bis 15 Gew.-% Phosphorsäure je Gewichtsmenge Siliciumdioxid und/oder $\gamma$-Aluminiumoxid. Die Phosphorsäure kann ganz oder teilweise als Phosphorpentoxid, Orthophosphorsäure, Pyrophosphorsäure oder Polyphosphorsäure, z.B. von 72 bis 88 Gew.-% Phosphorpentoxid, vorliegen und wird hier als Phosphorsäure berechnet, unabhängig von der tatsächlichen Konstitution der Phosphorsäure oder Phosphorsäureanhydrids.

Die Reaktion wird durchgeführt, indem man die auf Reaktionstemperatur erhitzten Ausgangsstoffe II und III über den auf Reaktionstemperatur erhitzten Katalysator in einem Festbettreaktor leitet. Die Verweilzeit beträgt in der Regel weniger als 0,5 Sekunden im Reaktionsraum. Das den Reaktionsraum verlassende Gemisch wird bei einer Temperatur oberhalb des Siedepunktes des 2-Imidazolins bei dem eingestellten Druck in eine Trennkolonne geleitet. In der Trennkolonne werden die Leichtsieder kontinuierlich abgetrennt. Im Kolonnensumpf fällt das 2-Imidazolin in einer Reinheit von über 99 % an.

Die nach dem Verfahren der Erfindung herstellbaren 2-Imidazoline I sind wertvolle Ausgangsstoffe bei der Herstellung von Farbstoffen, Pharmazeutika und pflanzenschutzmitteln. 2-Imidazoline werden als Katalysatoren für Polymerisationsreaktionen und Aldolkondensation eingesetzt.

Die in den folgenden Beispielen aufgeführten Teile bedeuten Gewichtsteile. Die Gewichtsteile verhalten sich zu Volumenteilen wie Kilogramm zu Liter.

Beispiele

Beispiel 1

111 Teile 1,2-Diaminopropan und 90 Teile Essigsäure werden stündlich getrennt in einen auf 180 °C erhitzten horizontalen Quarzverdampfer dosiert und der Dampf durch den auf 300 °C erhitzten Festbettreaktor geleitet. Der Festbettreaktor ist ein vertikales auf dem Verdampfer sitzendes, elektrisch beheizbares Quarzrohr von 35 mm Innendurchmesser, das nach unten mit einer eingemolzenen Quarzfritte abgeschlossen ist. Das Quarzrohr ist mit 100 Teilen eines Katalysators aus 5 Gew.-% $H_3PO_4$ und 95 Gew.-% $\gamma$-Aluminiumoxid gefüllt. Der den Reaktor verlassende Dampf wird direkt einer Trennkolonne zugeführt. Die Höhe der Kolonne beträgt 700 mm, der Durchmesser 30 mm. Gefüllt ist die Kolonne mit 10 Laborgewebepackungen der Fa. Sulzer; 3 oberhalb des Zulaufs, 7 unterhalb. Die Kolonne ist beheizt und mit einem magnetischen Rücklaufteiler versehen. Die gesamte Anlage wird so betrieben, daß der Druck in der Kolonne 100 mbar beträgt. Das 2,4-Dimethylimidazolin wird über den Sumpf abgelassen. Man erhält stündlich 143

Teile reines 2,4-Dimethylimidazolin (97 % der Theorie, bezogen auf eingesetzte Essigsäure).

Vergleichsbeispiel 1 analog EP-A-12 371, Beispiel 1

163 Teile 1,2-Diaminopropan und 120 Teile Essigsäure werden stündlich mit 5000 Volumenteilen Stickstoff in einen horizontalen Quarzverdampfer dosiert, bei 300 °C verdampft und durch den auf 300 °C erhitzten Wirbelreaktor geleitet. Der Wirbelreaktor ist ein vertikales auf dem Verdampfer sitzendes, elektrisch, beheizbares Quarzrohr, das nach unten mit einer eingeschmolzenen Quarzfritte abgeschlossen ist. Das Quarzrohr ist mit 200 Teilen eines Katalysators aus 5 Gew.-% $H_3PO_4$ und 95 Gew.-% $\gamma$-Aluminiumoxid zur Hälfte gefüllt. Die den Reaktor verlassenden Dämpfe werden kondensiert und das gesammelte Kondensat anschließend fraktioniert destilliert. Man erhält stündlich 203 Teile Kondensat. Die Destillation von 203 Teilen Kondensat ergibt 137 Teile reines 2,4-Dimethylimidazolin (70 % der Theorie bezogen auf eingesetzte Essigsäure).

Beispiel 2

Man verfährt wie in Beispiel 1, setzt jedoch 90 Teile 1,2-Diaminoethan und 90 Teile Essigsäure ein. Man erhält 118 Teile 2-Methylimidazolin (94 % bezogen auf eingesetzte Essigsäure).

Vergleichsbeispiel 2 analog EP-A-12 371, Beispiel 1

Man verfährt wie im Vergleichsbeispiel 1, setzt jedoch 132 Teile 1,2-Diaminoethan und 120 Teile Essigsäure ein. Man erhält stündlich 252 Teile Kondensat. Aus 252 Teilen Kondensat erhält man 104 Teile 2-Methylimidazolin (62 % der Theorie, bezogen auf eingesetzte Essigsäure).

Beispiel 3

Man verfährt wie in Beispiel 1, setzt jedoch 90 Teile 1,2-Diaminoethan und 111 Teile Propionsäure ein. Man erhält 143 Teile 2-Ethylimidazolin (97 % der Theorie, bezogen auf eingesetzte Propionsäure).

Vergleichsbeispiel 3 analog EP-A-12 371, Beispiel 1

Man verfährt wie in Vergleichsbeispiel 1, setzt jedoch 132 Teile 1,2-Diaminoethan und 148 Teile Propionsäure ein. Man erhält stündlich 210 Teile Kondensat. Aus 210 Teilen Kondensat erhält man 138 Teile 2-Ethylimidazolin (71 % der Theorie, bezogen auf eingesetzte Propionsäure).

**Ansprüche**

1. Verfahren zur Herstellung von 2-Imidazolinen der allgemeinen Formeln Ia und Ib

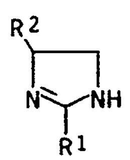

(Ia)

bzw.

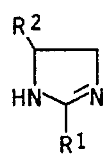

(Ib)

in denen $R^1$ $C_1$- bis $C_8$-Alkyl, $C_7$- bis $C_{12}$-Aralkyl oder Phenyl und $R^2$ Wasserstoff oder $C_1$- bis $C_8$-Alkyl bedeuten, durch Umsetzung von 1,2-Diaminen der allgemeinen Formel II

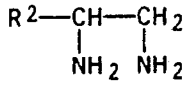

(II),

in der $R^2$ die obengenannte Bedeutung hat, mit Carbonsäuren der allgemeinen Formel III

$R^1$-$CO_2H$ (III), in der $R^1$ die obengenannte Bedeutung besitzt, in einem Verhältnis von 0,8 bis 1,2 mol Ausgangsstoff II je Mol Ausgangsstoff III, bei Temperaturen von 250 bis 400 °C in der Gasphase an Katalysatoren aus γ-Aluminiumoxid und/oder Siliciumoxid, welche gegebenenfalls mit Phophorsäure dotiert sind, dadurch gekennzeichnet, daß man die Reaktion im Festbett durchführt.

2. Verfahren nach Anspruch 1, dadurch gekennzeichnet, daß man den gasförmigen Reaktoraustrag ohne Zwischenkondensation kontinuierlich destilliert.

Europäisches Patentamt

**EUROPÄISCHER RECHERCHENBERICHT**

Nummer der Anmeldung

EP 90114237.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | DE - A1 - 2 854 428 (BASF) * Ansprüche 1,2 * | 1,2 | C 07 D 233/06 |
| D,A | DE - A1 - 2 615 886 (BASF) * Anspruch * | 1 | |
| A | DE - A1 - 2 701 372 (VEBA-CHEMIE) * Ansprüche 1-4 * | 1 | |
| A | CHEMICAL ABSTRACTS, Band 84, Nr. 3, 19. Jänner 1976, Columbus, Ohio, USA KITA, MASSAYOSHI et al. "Reaction and utilization of amines. III. Cyclization of long-chain N-acylethylene-diamines" Seite 410, Spalte 1, Zusammenfassung-Nr. 16 712d & Yukagaku 1975, 24(2), 112-15 | 1 | **RECHERCHIERTE SACHGEBIETE (Int Cl 5)** |
| | | | C 07 D 233/00 |
| A | CHEMICAL ABSTRACTS, Band 67, Nr. 15, 9. Oktober 1967, Columbus, Ohio, USA V.I. Isagulyants et al. "Ethylenediamine condensation with monocarboxylic acids" Seite 6929, Spalte 2, Zusammenfassung-Nr. 73 557e & Dokl. Akad. Nauk Arm. SSR 44(1), 23-8(1967) | 1 | |
| A | CHEMICAL ABSTRACTS, Band 73, Nr. 19, 9. November 1970, Columbus, Ohio, USA | 1 | |

Der vorliegende Recherchenbericht wurde für alle Patentansprüche erstellt

| Recherchenort | Abschlußdatum der Recherche | Prüfer |
|---|---|---|
| WIEN | 11-10-1990 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung



EP 90114237.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| | ISAGULYANTS, V.I. et al. "Catalytic method for synthesizing 2-alkyl imidazolines from carboxylic acid esters" Seite 368, Spalte 2, Zusammenfassung-Nr. 98 868u & Zh. Org. Khim. 1970, 6(8), 1755 -- | | |
| A | CHEMICAL ABSTRACTS, Band 86, Nr. 1, 3. Jänner 1977, Columbus, Ohio, USA PISKOV, V.B. et al. "Synthesis of 2-imidazolines in ethylene glycol" Seite 465, Spalte 2, Zusammenfassung-Nr. 5 372h & Khim. Geterotsikl. Soedin. 1976, (8), 1112-18 ---- | 1 | |

RECHERCHIERTE SACHGEBIETE (Int Cl⁵)

Der vorliegende Recherchenbericht wurde für alle Patentansprüche erstellt.

| Recherchenort | Abschlußdatum der Recherche | Prüfer |
|---|---|---|
| WIEN | 11-10-1990 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82